# EUROPEAN PATENT SPECIFICATION

(11) **EP 3 960 227 B1**
(45) Date of publication and mention of the grant of the patent: **07.10.2026**
(21) Application number: 20794073.5
(22) Date of filing: 22.04.2020
(51) Int. Cl.: A61M 35/00, B05B 17/06, A61M 11/00, A61M 15/00

(54) **ATOMIZATION DEVICE**
ZERSTÄUBUNGSVORRICHTUNG
DISPOSITIF D'ATOMISATION

(30) Priority: 23.04.2019 US 201962837209 P
(43) Date of publication of application: 02.03.2022
(73) Proprietor: Microbase Technology Corp., Taoyuan City 334 (TW)
(72) Inventor: HSIEH, Shu-pin, Taoyuan City 334 (TW); LIN, Chien-hua, Taoyuan City 334 (TW); LU, Chih-wei, Taoyuan City 334 (TW); HUNG, Chi-shan, Taoyuan City 334 (TW); WU, Jo-ling, Taoyuan City 334 (TW)
(74) Representative: Viering, Jentschura & Partner mbB Patent- und Rechtsanwälte
(86) International application number: PCT/CN2020/086162
(87) International publication number: WO 2020/216254

(56) References cited:
- WO-A1-2018/121813
- CN-A- 103 286 046
- CN-A- 106 139 337
- CN-A- 107 213 538
- CN-U- 201 970 828
- CN-U- 203 609 747
- CN-U- 203 609 747
- GB-A- 2 536 259
- US-A1- 2008 110 453
- US-A1- 2009 293 868
- US-A1- 2011 283 996

## Description

### FIELD OF THE DISCLOSURE

The present disclosure relates to an atomization device, and more particularly to an atomization device having a buffering chamber.

### BACKGROUND OF THE DISCLOSURE

CN203609747U discloses an atomization device using a disposable container. To-be-atomized liquid is filled in the disposable container, and one end of the disposable container is a closed mounting end. The atomization device comprises a body, an assembling ring, an atomization module and a cover body. After the disposable container is assembled on the assembling ring, a liquid storage space is formed. The liquid storage space is provided with a puncture pillar corresponding to the mounting end; the puncture pillar is in the shape of a polygonal pyramid, and a plurality of gas liquid exchange channels are formed in the surface of the puncture pillar; after the mounting end is punctured by the puncture pillar, the disposable container is extruded from the outside by a press part of the surface of the cover body, so that the to-be-atomized liquid flows into the liquid storage space through the gas liquid exchange channels, and then, is released, atomized and sprayed by the atomization module so as to greatly improve convenience during assembling and use.

An example of an atomizing unit with directly connectable ampoule is known from WO2018/121813 A1.

An example of an enhanced semi-automatic emergency medication dose nebulizer is known from US2011/283996 A1.

An example of an inhalation therapy device comprising an ampoule for storing a medicament to be nebulized is known from US2009/293868 A1.

An example of a nebulizer and methods for controlling the nebulizer is known from US2008/110453 A1.

A conventional atomization device includes a liquid supply container and an atomizing module that is connected to the liquid supply container. The liquid supply container is configured to store liquid that can be transferred from the liquid supply container to the atomizing module for an atomizing process. However, in the conventional atomization device, the liquid supply container is directly connected to the atomizing module, and the atomizing process is continuously implemented until the liquid stored in the liquid supply container is exhausted, which causes inconveniences in use.

### SUMMARY OF THE DISCLOSURE

In response to the above-referenced technical inadequacy, the present disclosure provides an atomization device to effectively improve on the issues associated with conventional atomization devices according to independent claim 1. The dependent claims show further embodiments of claim 1.

In one aspect, the present disclosure provides an atomization device, which includes a disposable liquid supply module, a carrier, and an atomizing module. The disposable liquid supply module includes a liquid storing member and an opener. The liquid storing member stores a liquid therein. The opener is configured to form an opening on the liquid storing member. The carrier is detachably assembled to the disposable liquid supply module. The atomizing module includes a vibrator assembled to the carrier and a microporous film that is assembled to the disposable liquid supply module and that detachably abuts against the vibrator. The disposable liquid supply module has a buffering chamber arranged between the atomizing module and the opening of the liquid storing member, and a volume of the buffering chamber is less than a volume of the liquid stored in the liquid storing member. The disposable liquid supply module is configured to be pressed so that an external force is exerted onto the liquid storing member and an inner pressure of the liquid storing member is changed, such that a part of the liquid is driven to flow from the opening into the buffering chamber for an atomizing process of the atomizing module. The opener is configured to be separated from the liquid storing member by being moved relative to the liquid storing member so as to form the opening on the liquid storing member. The disposable liquid supply module further includes a container having the buffering chamber therein, and the liquid storing member is inserted into the container, so that the opening is in spatial communication with the buffering chamber. The container has an inlet for an insertion of the liquid storing member and an outlet that corresponds in position to the atomizing module. The opener is configured to be separated from the liquid storing member by being retained in the inlet and being rotated relative to the liquid storing member, so that the opener that is separated from the liquid storing member is located in the buffering chamber. The microporous film is fixed to the container, and the microporous film covers the outlet.

In certain embodiments, the container includes a barrier arranged in the buffering chamber and located between the inlet and the outlet, and the opener separated from the liquid storing member is disposed on the barrier by passing through the inlet.

In certain embodiments, the opening is greater than 0 mm, and is not greater than 8 mm.

Therefore, in the atomization device provided by the present disclosure, the buffering chamber is jointly defined by the atomizing module and the carrier, and the volume of the buffering chamber is less than the volume of the liquid stored in the liquid storing member. Accordingly, the buffering chamber can receive the liquid stored in the liquid storing member over multiple times, such that the atomization device can atomize the liquid over multiple times through the buffering chamber.

The invention is set out in the appended set of claims.

These and other aspects of the present disclosure will become apparent from the following description of the embodiment taken in conjunction with the following drawings and their captions, although variations and modifications therein may be affected without departing from the scope of the novel concepts of the disclosure.

### BRIEF DESCRIPTION OF THE DRAWINGS

The described embodiments may be better understood by reference to the following description and the accompanying drawings, in which:
FIG. 1 is an exploded view of an atomization device according to a first embodiment of the present disclosure;
FIG. 2 is an assembled view of the atomization device according to the first embodiment of the present disclosure;
FIG. 3 is a cross-sectional view taken along line III-III of FIG. 2;
FIG. 4 shows an enlarged view of part IV of FIG. 3;
FIG. 5 is an exploded view of the atomization device according to a second embodiment of the present disclosure;
FIG. 6 is a perspective view of the atomization device according to the second embodiment of the present disclosure;
FIG. 7 is a perspective view showing the atomization device of FIG. 6 after an opener is removed from a liquid storing member;
FIG. 8 is a cross-sectional view taken along line VIII-VIII of FIG. 7;
FIG. 9 is a cross-sectional view of the atomization device according to a third embodiment of the present disclosure;
FIG. 10 is a cross-sectional view showing the atomization device of FIG. 9 after the opener is separated from the liquid storing member;
FIG. 11 is a cross-sectional view showing the atomization device of FIG. 10 after the opener is reassembled to the liquid storing member;
FIG. 12 is a perspective view of a disposable liquid supply module and a microporous film according to the third embodiment of the present disclosure;
FIG. 13 is a perspective view showing the disposable liquid supply module and the microporous film of FIG. 12 after the opener is separated from the liquid storing member;
FIG. 14 is a perspective view showing the disposable liquid supply module and the microporous film of FIG. 13 after the opener is reassembled to the liquid storing member; and
FIG. 15 is a cross-sectional view taken along line XV-XV of FIG. 14.

### DETAILED DESCRIPTION OF THE EXEMPLARY EMBODIMENTS

The second embodiment described herein represents an embodiment of the invention as defined in the appended claims. The first and third embodiments are examples useful for understanding but not forming part of the invention.

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a", "an", and "the" includes plural reference, and the meaning of "in" includes "in" and "on". Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first", "second" or "third" can be used to describe various components, signals or the like, which are for distinguishing one component/signal from another one only, and are not intended to, nor should be construed to impose any substantive limitations on the components, signals or the like.

### [First Embodiment]

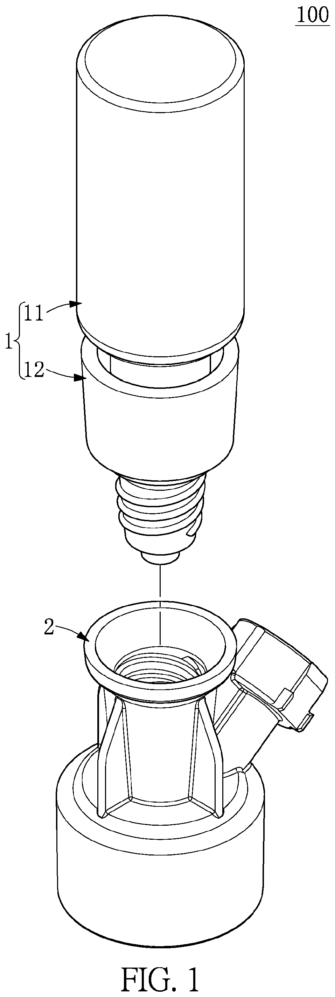

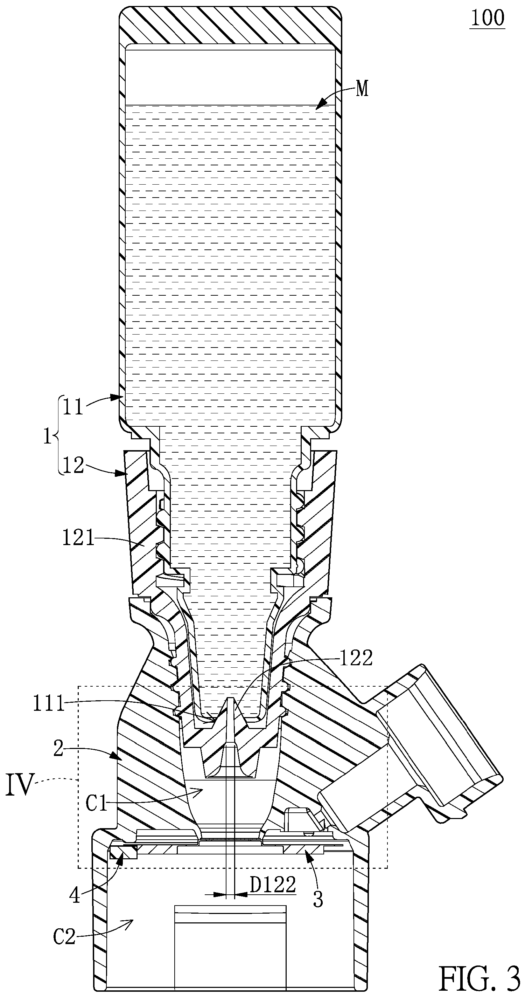

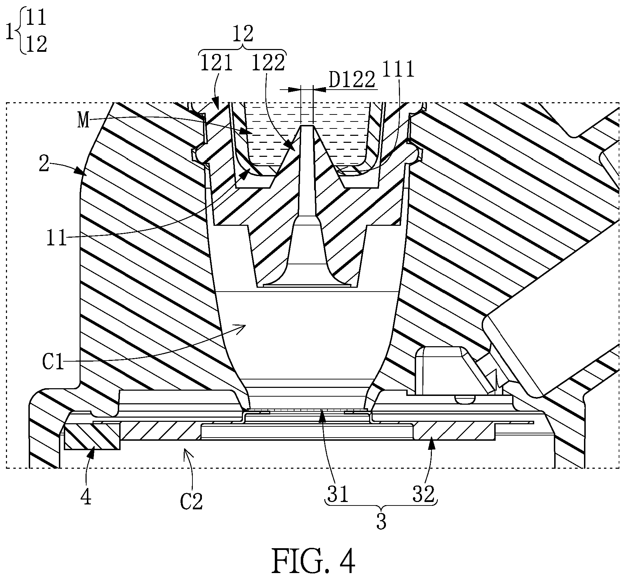

Referring to FIG. 1 to FIG. 4, a first embodiment of the present disclosure provides an atomization device 100. The atomization device 100 of the present embodiment includes a disposable liquid supply module 1, a carrier 2 detachably assembled to the disposable liquid supply module 1, and an atomizing module 3 that is assembled to at least one of the disposable liquid supply module 1 and the carrier 2. In the present embodiment, the atomizing module 3 is exemplified as being only assembled to the carrier 2.

The disposable liquid supply module 1 includes a liquid storing member 11 and an opener 12 that is disposed on the liquid storing member 11, and the opener 12 is configured to form an opening 111 on the liquid storing member 11, but the present disclosure is not limited thereto. For example, in other embodiments of the present disclosure not shown in the drawings, the liquid storing member 11 and the opener 12 can be two components that are separate from each other; or, the opener 12 can be disposed on the carrier 2. In other words, the opener 12 can be disposed on at least one of the liquid storing member 11 and the carrier 2.

Specifically, the liquid storing member 11 stores liquid M (e.g., a liquid medicine or a skincare liquid) therein, and the type of the liquid M can be adjusted or changed according to design requirements. It should be noted that the liquid storing member 11 in the present embodiment might be a disposable plastic object that is elongated and elastically pressable. Further, the liquid storing member 11 cannot be refilled with the liquid M. In other words, the liquid storing member 11 is pre-filled with the liquid M. In addition, the shape of the liquid storing member 11 can be adjusted or changed according to design requirements and is not limited to the drawings of the present embodiment.

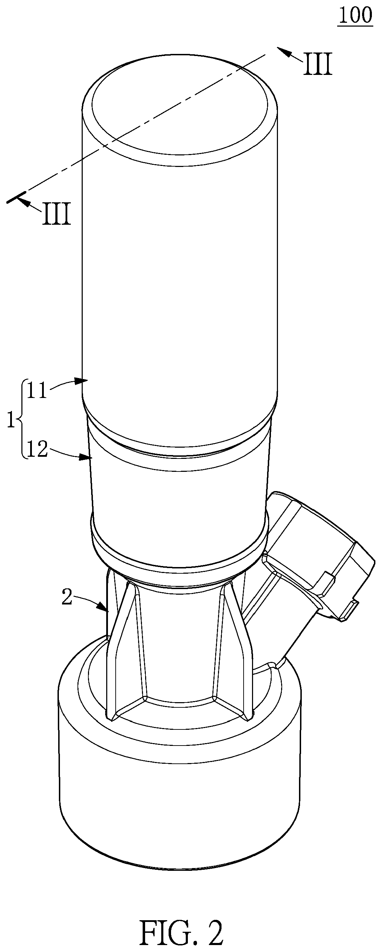

The opener 12 is disposed on an outer surface of the liquid storing member 11, and includes a cover 121 and a piercing tube 122 that is connected to a center portion of the cover 121. The opener 12 is movable relative to the liquid storing member 11 from an initial position to a use position (as shown in FIG. 2). When the opener 12 is at the initial position, the liquid storing member 11 is a sealed structure. When the opener 12 is at the use position, the piercing tube 122 pierces into the liquid storing member 11, so as to form the opening 111 on the liquid storing member 11.

It should be noted that the opener 12 in the present embodiment is rotatably disposed on the outer surface of the liquid storing member 11 through the cover 121, so that the opener 12 is rotatable relative to the liquid storing member 11 between the initial position and the use position. Accordingly, through an operation mechanism of the opener 12 and the liquid storing member 11 of the disposable liquid supply module 1, when the opening 111 of the liquid storing member 11 is formed by the opener 12, the opening 111 and an adjacent portion of the liquid storing member 11 can effectively avoid being touched by a user, thereby reducing a probability of polluting the liquid M.

Moreover, an inner diameter D122 of the piercing tube 122 can be controlled to be greater than 0 mm and less than or equal to 8 mm, so that when no external force is exerted upon the liquid storing member 11, the liquid M in the liquid storing member 11 does not easily flow outside of the liquid storing member 11 along the piercing tube 122. Preferably, the inner diameter D122 of the piercing tube 122 is not less than 1 mm but not greater than 4.5 mm.

The carrier 2 is detachably assembled to the opener 12 of the disposable liquid supply module 1. In other words, the carrier 2 is indirectly and detachably assembled to the liquid storing member 11 through the opener 12. Moreover, the atomizing module 3 is assembled to the carrier 2, and the atomization device 100 has a buffering chamber C1 arranged between the atomizing module 3 and the opening 111 of the liquid storing member 11. That is, the atomizing module 3 and the carrier 2 in the present embodiment jointly form (or define) the buffering chamber C1 that corresponds in position to the piercing tube 122.

Specifically, the carrier 2 also has an atomizing chamber C2, the atomizing module 3 is located between the atomizing chamber C2 and the buffering chamber C1, and a volume of the buffering chamber C1 is less than a volume of the liquid M stored in the liquid storing member 11, so that the buffering chamber C1 can receive the liquid M stored in the liquid storing member 11 over multiple times.

Furthermore, the atomizing module 3 includes a microporous film 31 and a vibrator 32 that (directly or indirectly) abuts against the microporous film 31. In the present embodiment, the microporous film 31 and the vibrator 32 of the atomizing module 3 are assembled to the carrier 2, and the microporous film 31 is configured to separate the atomizing chamber C2 and the buffering chamber C1 from each other. A substantially central portion of the microporous film 31 has a plurality of atomizing holes penetrating therethrough, and a quantity of the atomizing holes and the shape of any one of the atomizing holes can be changed according to a design-required particle size, but the present disclosure is not limited thereto. Moreover, the vibrator 32 in the present embodiment is a piezoelectric (PZT) sheet that has an annular shape, and a center hole of the vibrator 32 corresponds in position to the atomizing holes of the microporous film 31. The microporous film 31 is made of at least one of or a combination of polymers that are selected from polyimide (PI), polyethylene (PE), polypropylene (PP), and polyetheretherketone (PEEK).

Accordingly, when the opener 12 is at the use position, the disposable liquid supply module 1 is configured to be pressed so that an external force is exerted onto the liquid storing member 11 and an inner pressure of the liquid storing member 11 is changed, such that a part of the liquid M is driven to flow from the piercing tube 122 (or the opening 111) into the buffering chamber C1 for an atomizing process of the atomizing module 3. Moreover, the atomizing process of the atomizing module 3 refers to having the part of the liquid M in the buffering chamber C1 be atomized toward the atomizing chamber C2 through the atomizing holes of the microporous film 31 when the vibrator 32 vibrates the microporous film 31.

It should be noted that the disposable liquid supply module 1 in the present embodiment is pressed by the user's finger, but the present disclosure is not limited thereto. For example, in other embodiments of the present disclosure not shown in the drawings, the disposable liquid supply module 1 can be pressed by a mechanical manner, so as to cause gas to squeeze an interior of the disposable liquid supply module 1.

In addition, the atomization device 100 can further include a pressure sensor 4 that is electrically coupled to the atomizing module 3 (e.g., the vibrator 32 of the atomizing module 3) according to design requirements. The pressure sensor 4 is configured to detect a pressure of the atomizing chamber C2, so that when the pressure of the atomizing chamber C2 is lower than a predetermined value, the pressure sensor 4 can drive the atomizing module 3 to atomize the part of the liquid M in the buffering chamber C1.

### [Second Embodiment]

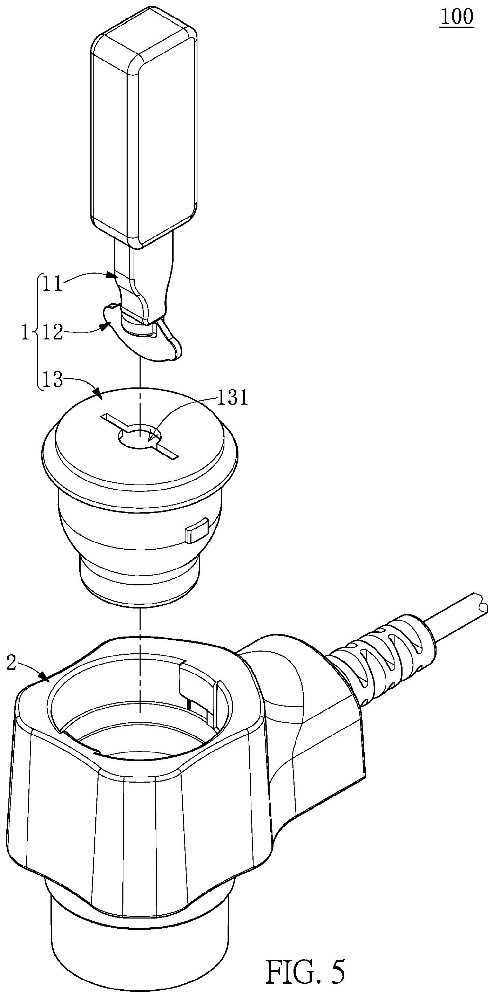

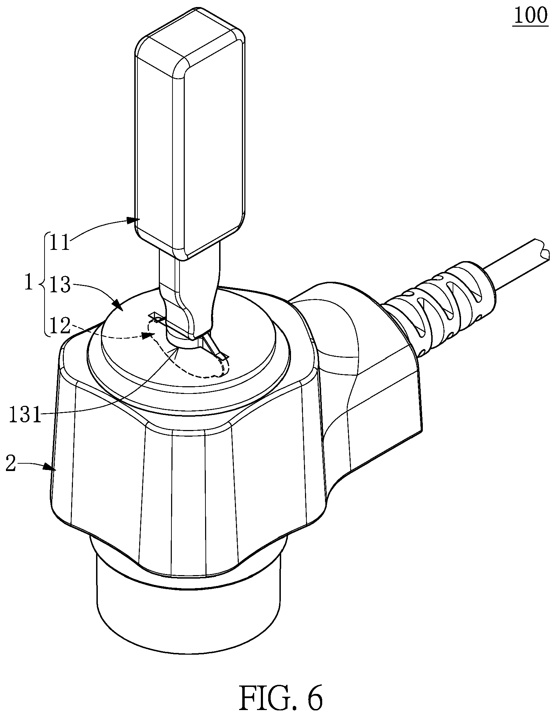

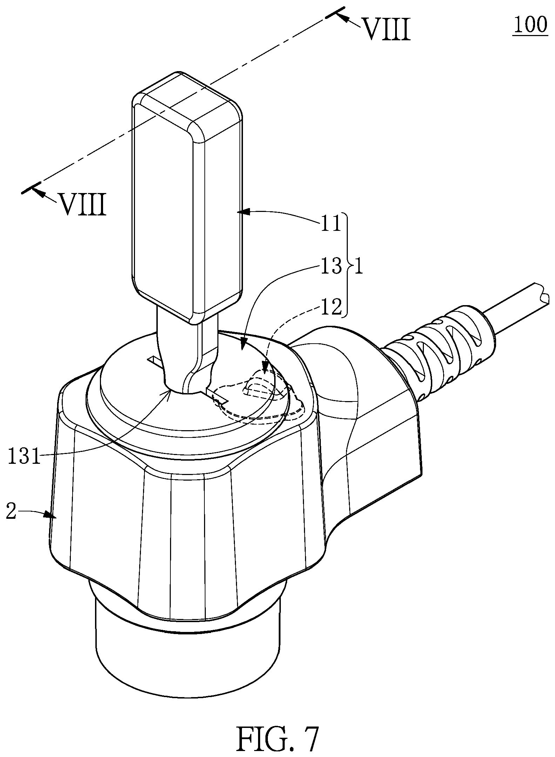

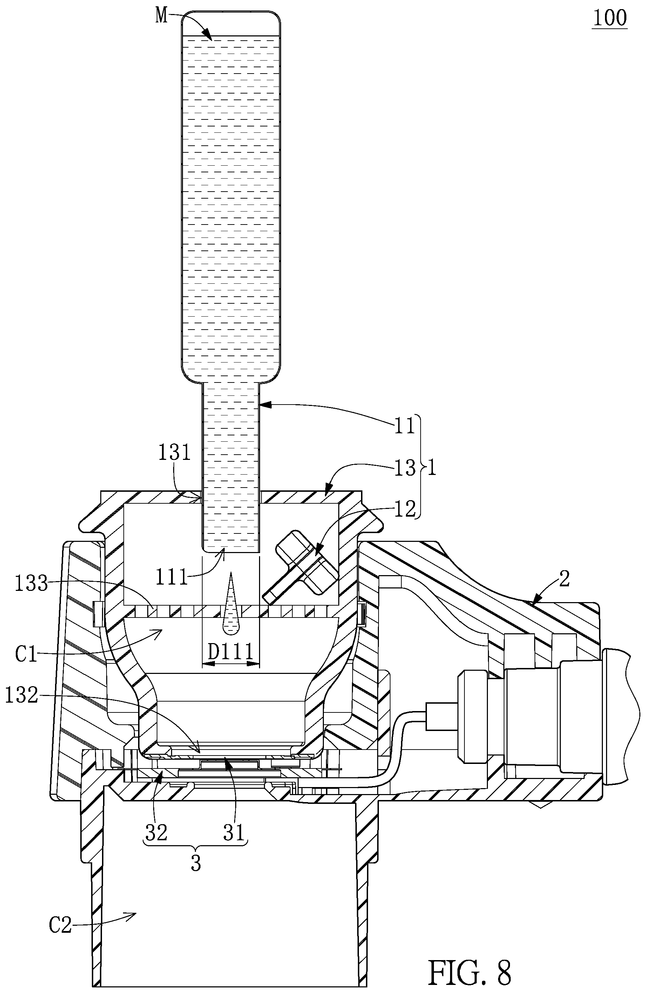

Referring to FIG. 5 to FIG. 8, a second embodiment of the present disclosure provides an atomization device 100, which includes a disposable liquid supply module 1, a carrier 2 detachably assembled to the disposable liquid supply module 1, and an atomizing module 3 that is assembled to at least one of the disposable liquid supply module 1 and the carrier 2. In the present embodiment, the atomizing module 3 is exemplified as being assembled to the disposable liquid supply module 1 and the carrier 2.

The disposable liquid supply module 1 includes a liquid storing member 11, an opener 12 that is configured to form an opening 111 on the liquid storing member 11, and a container 13 that is spaced apart from the liquid storing member 11 and the opener 12. Specifically, the liquid storing member 11 stores liquid M (e.g., a liquid medicine or a skincare liquid) therein, and the type of the liquid M can be adjusted or changed according to design requirements. It should be noted that the liquid storing member 11 in the present embodiment is an elongated and elastically pressable object, and might be a disposable plastic object. Further, the liquid storing member 11 cannot be refilled with the liquid M. In addition, the shape of the liquid storing member 11 can be adjusted or changed according to design requirements and is not limited to the drawings of the present embodiment.

The opener 12 is configured to be separated from the liquid storing member 11 by being moved relative to the liquid storing member 11, so as to form the opening 111 on the liquid storing member 11. In the present embodiment, the opener 12 is integrally connected to the liquid storing member 11, and a connection interface between the opener 12 and the liquid storing member 11 is easily broken, so that when the opener 12 is rotated relative to the liquid storing member 11, the opener 12 is separated from the liquid storing member 11 through the connection interface. Accordingly, the connection interface is formed to have the opening 111.

Moreover, the opening 111 of the liquid storing member 11 can be controlled to be greater than 0 and not greater than 8 mm, so that when no external force is exerted upon the liquid storing member 11, the liquid M in the liquid storing member 11 does not easily flow outside of the liquid storing member 11 through the opening 111. The opening 111 of the liquid storing member 11 is preferably not less than 1 mm and not greater than 4.5 mm.

The container 13 has a buffering chamber C1 therein, and a volume of the buffering chamber C1 is less than a volume of the liquid M stored in the liquid storing member 11, so that the buffering chamber C1 can receive the liquid M stored in the liquid storing member 11 over multiple times. Moreover, the container 13 has an inlet 131 and an outlet 132. The inlet 131 and the outlet 132 are in spatial communication with the buffering chamber C1, and the inlet 131 and the outlet 132 in the present embodiment are arranged on two opposite sides of the buffering chamber C1, respectively.

The inlet 131 corresponds in shape to the opener 12. The opener 12 is configured to be separated from the liquid storing member 11 by being retained in the inlet 131 and being rotated relative to the liquid storing member 11, such that the opening 111 is formed on the liquid storing member 11. Accordingly, through an operation mechanism of the opener 12 and the container 13 of the disposable liquid supply module 1, when the opening 111 of the liquid storing member 11 is formed by the opener 12, the opening 111 and an adjacent portion of the liquid storing member 11 can effectively avoid being touched by a user, thereby reducing a probability of polluting the liquid M.

Specifically, after the opener 12 is separated from the liquid storing member 11, the opener 12 is located in the buffering chamber C1, and the liquid storing member 11 is inserted into the container 13 through the inlet 131, so that the opening 111 is in spatial communication with the buffering chamber C1. In the present embodiment, the container 13 includes a barrier 133 arranged in the buffering chamber C1 and located between the inlet 131 and the outlet 132, so that the opener 12 separated from the liquid storing member 11 is disposed on the barrier 133 by passing through the inlet 131.

The opening 111 of the liquid storing member 11 is preferably adjacent to the barrier 133 and is located at one side of the opener 12, thereby preventing the liquid M that flows outside of the liquid storing member 11 through the opening 111 from contacting the opener 12, but the present disclosure is not limited thereto. For example, in other embodiments of the present disclosure not shown in the drawings, a portion of the liquid storing member 11 having the opening 111 can be inserted into the barrier 133, thereby preventing the liquid M that flows outside of the liquid storing member 11 through the opening 111 from contacting the opener 12.

The carrier 2 is detachably assembled to the container 13 of the disposable liquid supply module 1. In other words, the carrier 2 is indirectly and detachably assembled to the liquid storing member 11 through the container 13. Moreover, the atomizing module 3 is assembled to the carrier 2, the outlet 132 of the container 13 corresponds in position to (and faces toward) the atomizing module 3, and the buffering chamber C1 is arranged between the atomizing module 3 and the opening 111 of the liquid storing member 11.

Specifically, the carrier 2 further has an atomizing chamber C2 therein, and the atomizing module 3 is located between the atomizing chamber C2 and the buffering chamber C1. The atomizing module 3 includes a microporous film 31 and a vibrator 32 that detachably abuts against the microporous film 31. In the present embodiment, the microporous film 31 of the atomizing module 3 is fixed to the container 13 and covers the outlet 132, so as to separate the atomizing chamber C2 and the buffering chamber C1 from each other. The vibrator 32 of the atomizing module 3 is assembled to the carrier 2. In addition, according to the first and second embodiments, the atomizing module 3 of the present disclosure can be assembled to at least one of the liquid storing member 11 and the carrier 2.

The microporous film 31 has a plurality of atomizing holes penetrating a substantial center portion thereof, and a quantity of the atomizing holes and the shape of any one of the atomizing holes can be changed according to a design-required particle size, but the present disclosure is not limited thereto. Moreover, the vibrator 32 in the present embodiment is a piezoelectric (PZT) sheet being in an annular shape, and a center hole of the vibrator 32 corresponds in position to the atomizing holes of the microporous film 31.

Accordingly, the disposable liquid supply module 1 is configured to be pressed so that an external force is exerted onto the liquid storing member 11 and an inner pressure of the liquid storing member 11 is changed, such that a part of the liquid M is driven to flow from the opening 111 of the liquid storing member 11 into the buffering chamber C1 for an atomizing process of the atomizing module 3. Moreover, the atomizing process of the atomizing module 3 refers to having the part of the liquid M in the buffering chamber C1 be atomized toward the atomizing chamber C2 through the atomizing holes of the microporous film 31 when the vibrator 32 vibrates the microporous film 31.

It should be noted that the disposable liquid supply module 1 in the present embodiment is pressed by the user's finger, but the present disclosure is not limited thereto. For example, in other embodiments of the present disclosure not shown in the drawings, the disposable liquid supply module 1 can be pressed by a mechanical manner, so as to cause gas to squeeze an interior of the disposable liquid supply module 1.

In addition, in other embodiments of the present disclosure not shown in the drawings, the atomization device 100 can further include a pressure sensor that is electrically coupled to the atomizing module 3 (e.g., the vibrator 32 of the atomizing module 3) according to design requirements. The function of the pressure sensor of the present embodiment is identical to that of the first embodiment, and will not be reiterated herein.

### [Third Embodiment]

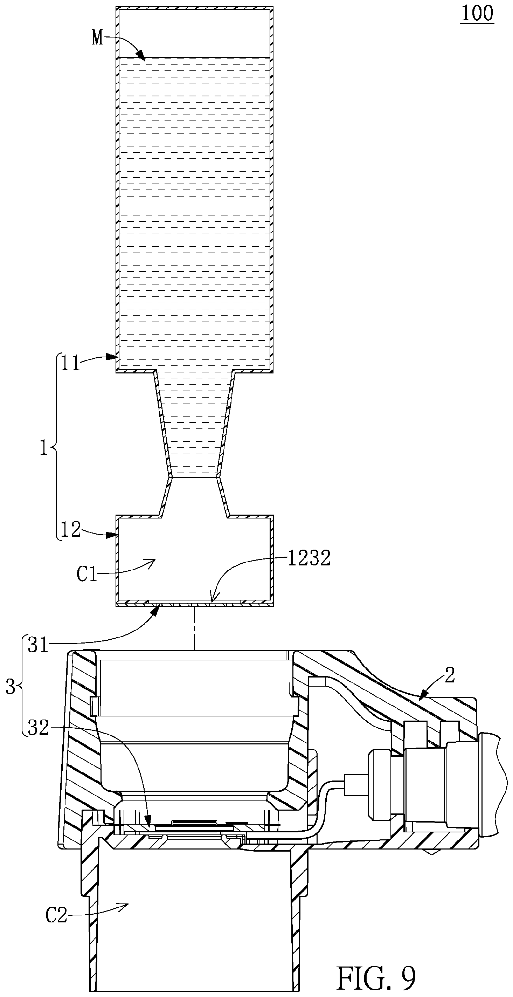

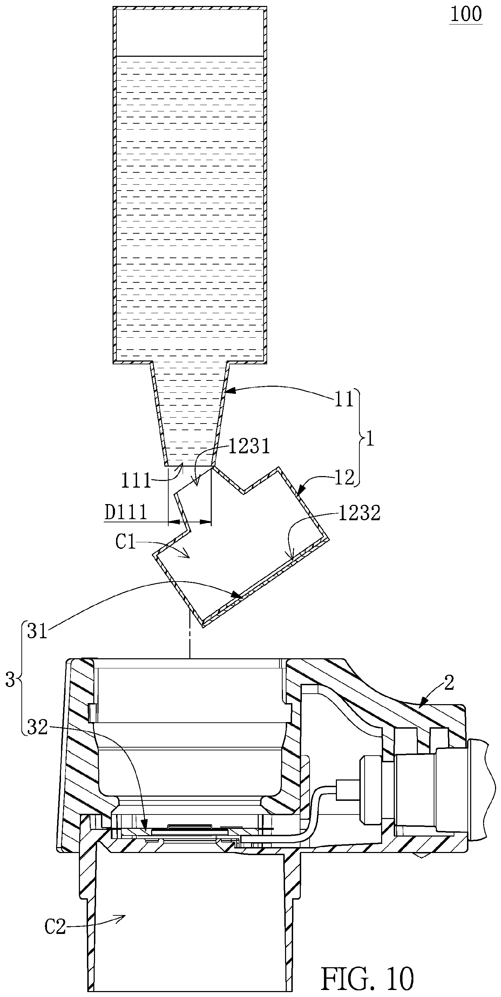

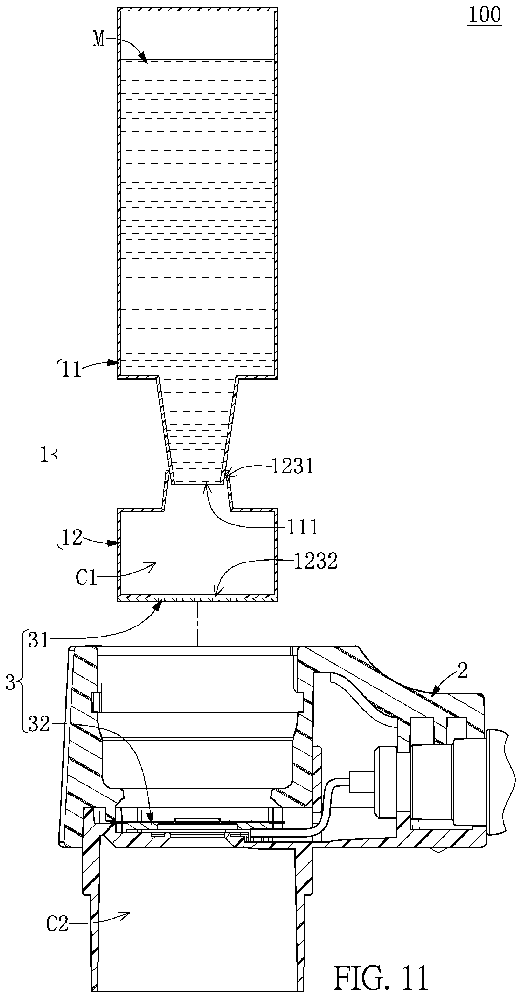

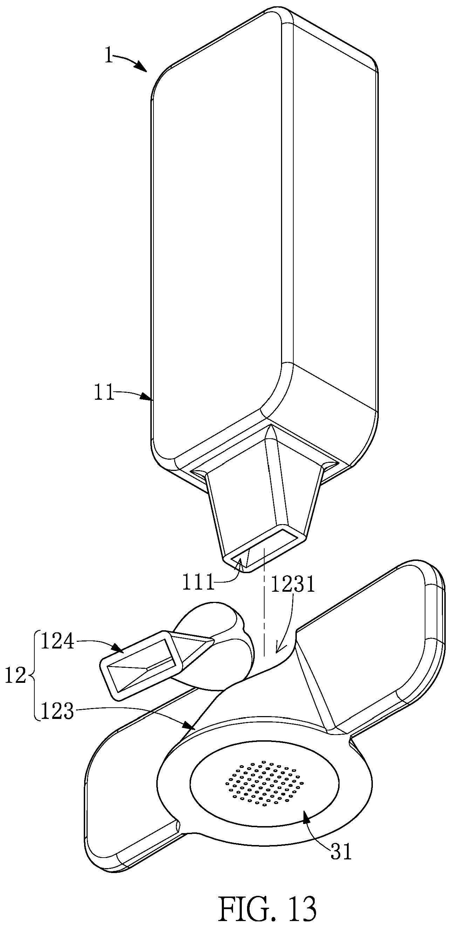

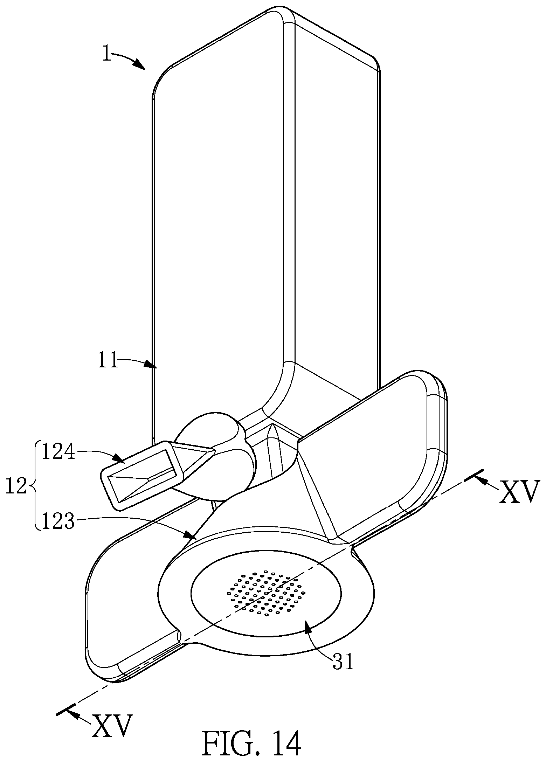

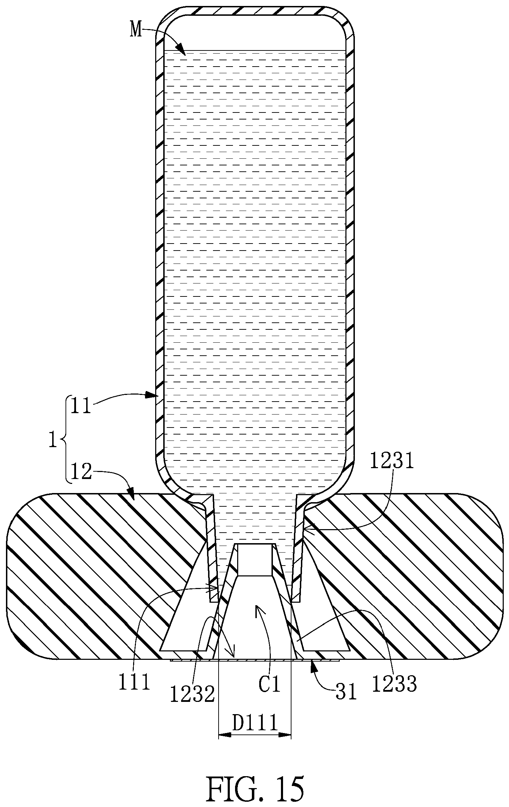

Referring to FIG. 9 to FIG. 15, a third embodiment of the present disclosure provides an atomization device 100. As shown in FIG. 9 to FIG. 11, the atomization device 100 includes a disposable liquid supply module 1, a carrier 2 detachably assembled to the disposable liquid supply module 1, and an atomizing module 3 that is assembled to at least one of the disposable liquid supply module 1 and the carrier 2. In the present embodiment, the atomizing module 3 is exemplified as being assembled to the disposable liquid supply module 1 and the carrier 2.

It should be noted that the carrier 2 and the atomizing module 3 of the present embodiment are similar to those of the second embodiment. Therefore, for the features of the carrier 2 and the atomizing module 3 of the present embodiment, reference can also be made to FIG. 5 to FIG. 8 of the second embodiment.

The disposable liquid supply module 1 includes a liquid storing member 11 and an opener 12 that is configured to form an opening 111 on the liquid storing member 11. Specifically, the liquid storing member 11 stores liquid M (e.g., a liquid medicine or a skincare liquid) therein, and the type of the liquid M can be adjusted or changed according to design requirements. It should be noted that the liquid storing member 11 in the present embodiment is an elongated and elastically pressable object, and might be a disposable plastic object. Further, the liquid storing member 11 cannot be refilled with the liquid M. In addition, the shape of the liquid storing member 11 can be adjusted or changed according to design requirements and is not limited to the drawings of the present embodiment.

The opener 12 is configured to be separated from the liquid storing member 11 by being moved relative to the liquid storing member 11, so as to form the opening 111 on the liquid storing member 11. In the present embodiment, the opener 12 is integrally connected to the liquid storing member 11, and a connection interface between the opener 12 and the liquid storing member 11 is easily broken, so that when the opener 12 is moved relative to the liquid storing member 11, the opener 12 is separated from the liquid storing member 11 through the connection interface. Accordingly, the connection interface is formed to have the opening 111.

It should be noted that the opening 111 of the liquid storing member 11 can be controlled to be greater than 0 and not greater than 8 mm, so that when no external force is exerted upon the liquid storing member 11, the liquid M in the liquid storing member 11 does not easily flow outside of the liquid storing member 11 through the opening 111. The opening 111 of the liquid storing member 11 is preferably not less than 1 mm and not greater than 4.5 mm.

Moreover, the opener 12 has a buffering chamber C1 therein, and a volume of the buffering chamber C1 is less than a volume of the liquid M stored in the liquid storing member 11, so that the buffering chamber C1 can receive the liquid M stored in the liquid storing member 11 over multiple times. Furthermore, the opener 12 has an inlet 1231 and an outlet 1232. The inlet 1231 and the outlet 1232 are in spatial communication with the buffering chamber C1, and the inlet 1231 and the outlet 1232 in the present embodiment are arranged on two opposite sides of the buffering chamber C1, respectively. Specifically, after the opener 12 is separated from the liquid storing member 11, one of the inlet 1231 of the opener 12 and the opening 111 of the liquid storing member 11 is inserted into the other one of the inlet 1231 of the opener 12 and the opening 111 of the liquid storing member 11, so that the opening 111 is in spatial communication with the buffering chamber C1.

Accordingly, through an operation mechanism of the opener 12 and the liquid storing member 11 of the disposable liquid supply module 1, when the opening 111 of the liquid storing member 11 is formed by the opener 12, the opening 111 and an adjacent portion of the liquid storing member 11 can effectively avoid being touched by a user, thereby reducing a probability of polluting the liquid M.

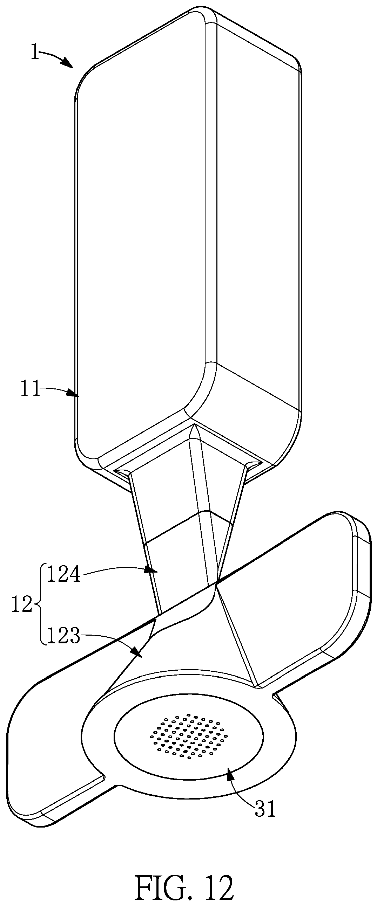

As shown in FIG. 12 to FIG. 15, in a specific structural configuration of the disposable liquid supply module 1 that satisfies the above technical features, the opener 12 includes a container portion 123 and a pre-connection portion 124 that is rotatably connected to the container portion 123. The container portion 123 has the inlet 1231 and the outlet 1232. The pre-connection portion 124 is integrally connected to the liquid storing member 11, so that the pre-connection portion 124 is configured to be separated from the liquid storing member 11 by being rotated (or moved) relative to the container portion 123, so as to form the opening 111 on the liquid storing member 11 and to form the inlet 1231 on the container portion 123.

Moreover, the opener 12 can have a piercing tube 1233 formed in the container portion 123. The piercing tube 1233 defines the buffering chamber C1 therein, an end of the piercing tube 1233 is in spatial communication with the outlet 1232, and another end of the piercing tube 1233 does not protrude from the inlet 1231. When the opening 111 of the liquid storing member 11 is inserted into the opener 12, the another end of the piercing tube 1233 is coupled to the opening 111 of the liquid storing member 11 and is gaplessly connected to the opening 111 of the liquid storing member 11, but the present disclosure is not limited thereto.

As shown in FIG. 9 to FIG. 15, the carrier 2 is detachably assembled to the opener 12 (e.g., the container portion 123 of the opener 12). In other words, the carrier 2 is indirectly and detachably assembled to the liquid storing member 11 through the opener 12. Moreover, the atomizing module 3 is assembled to the carrier 2, the outlet 1232 of the opener 12 corresponds in position to (and faces toward) the atomizing module 3, and the buffering chamber C1 is arranged between the atomizing module 3 and the opening 111 of the liquid storing member 11.

Specifically, the carrier 2 further has an atomizing chamber C2 therein, and the atomizing module 3 is located between the atomizing chamber C2 and the buffering chamber C1. The atomizing module 3 includes a microporous film 31 and a vibrator 32 that detachably abuts against the microporous film 31. In the present embodiment, the microporous film 31 of the atomizing module 3 is fixed to the opener 12 (e.g., the container portion 123 of the opener 12) and covers the outlet 1232, so as to separate the atomizing chamber C2 and the buffering chamber C1 from each other. The vibrator 32 of the atomizing module 3 is assembled to the carrier 2.

The microporous film 31 has a plurality of atomizing holes penetrating a substantial center portion thereof, and a quantity of the atomizing holes and the shape of any one of the atomizing holes can be changed according to a design-required particle size, but the present disclosure is not limited thereto. Moreover, the vibrator 32 in the present embodiment is a piezoelectric (PZT) sheet that has an annular shape, and a center hole of the vibrator 32 corresponds in position to the atomizing holes of the microporous film 31.

Accordingly, the disposable liquid supply module 1 is configured to be pressed so that an external force is exerted onto the liquid storing member 11 and an inner pressure of the liquid storing member 11 is changed, such that a part of the liquid M is driven to flow from the opening 111 into the buffering chamber C1 for an atomizing process of the atomizing module 3. Moreover, the atomizing process of the atomizing module 3 refers to having the part of the liquid M in the buffering chamber C1 can be atomized toward the atomizing chamber C2 through the atomizing holes of the microporous film 31 when vibrator 32 vibrates the microporous film 31.

It should be noted that the disposable liquid supply module 1 in the present embodiment is pressed by the user's finger, but the present disclosure is not limited thereto. For example, in other embodiments of the present disclosure not shown in the drawings, the disposable liquid supply module 1 can be pressed by a mechanical manner, so as to cause gas to squeeze an interior of the disposable liquid supply module 1.

In addition, in other embodiments of the present disclosure not shown in the drawings, the atomization device 100 can further include a pressure sensor that is electrically coupled to the atomizing module 3 (e.g., the vibrator 32 of the atomizing module 3) according to design requirements. The function of the pressure sensor of the present embodiment is identical to that of the first embodiment, and will not be reiterated herein.

### [Beneficial Effects of the Embodiments]

In conclusion, the atomization device provided by the present disclosure, the buffering chamber is jointly defined by the atomizing module and the carrier, and the volume of the buffering chamber is less than the volume of the liquid stored in the liquid storing member. Accordingly, the buffering chamber can receive the liquid stored in the liquid storing member over multiple times, such that the atomization device can atomize the liquid over multiple times through the buffering chamber.

Moreover, in the atomization device provided by the present disclosure, through the opening of the liquid storing member being formed by the opener, the opening and an adjacent portion of the liquid storing member can effectively avoid being touched by a user, thereby reducing a probability of polluting the liquid.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its scope.

## Claims

1. An atomization device (100), comprising:
a disposable liquid supply module (1) including:
a liquid storing member (11) that stores a liquid (M) therein; and
an opener (12) configured to form an opening (111) on the liquid storing member (11);
a carrier (2) detachably assembled to the disposable liquid supply module (1); and
an atomizing module (3) including a vibrator (32) assembled to the carrier (2) and a microporous film (31) that is assembled to the disposable liquid supply module (1) and that detachably abuts against the vibrator (32);
wherein the disposable liquid supply module (1) has a buffering chamber (C1) arranged between the atomizing module (3) and the opening (111) of the liquid storing member (11), and a volume of the buffering chamber (C1) is less than a volume of the liquid (M) stored in the liquid storing member (11);
wherein the disposable liquid supply module (1) is configured to be pressed so that an external force is exerted onto the liquid storing member (11) and an inner pressure of the liquid storing member (11) is changed, such that a part of the liquid (M) is driven to flow from the opening (111) into the buffering chamber (C1) for an atomizing process of the atomizing module (3);
wherein the opener (12) is configured to be separated from the liquid storing member (11) by being moved relative to the liquid storing member (11) so as to form the opening (111) on the liquid storing member (11), and wherein the disposable liquid supply module (1) further includes a container (13) having the buffering chamber (C1) therein, and the liquid storing member (11) is inserted into the container (13), so that the opening (111) is in spatial communication with the buffering chamber (C1);
wherein the container (13) has an inlet (131) for an insertion of the liquid storing member (11) and an outlet (132) that corresponds in position to the atomizing module (3), and wherein the opener (12) is configured to be separated from the liquid storing member (11) by being retained in the inlet (131) and being rotated relative to the liquid storing member (11), so that the opener (12) that is separated from the liquid storing member (11) is located in the buffering chamber (C1);
wherein the microporous film (31) is fixed to the container (13), and the microporous film (31) covers the outlet (132).

2. The atomization device according to claim 1, wherein the container (13) includes a barrier (133) arranged in the buffering chamber (C1) and located between the inlet (131) and the outlet (132), and the opener (12) that is separated from the liquid storing member (11) is disposed on the barrier (133) by passing through the inlet (131).

3. The atomization device according to claim 1, wherein the opening (111) is greater than 0 mm, and is not greater than 8 mm.

## Patentansprüche

1. Zerstäubungsvorrichtung (100), welche aufweist:
ein Einweg-Flüssigkeit-Zufuhr-Modul (1), welches aufweist:
ein Flüssigkeit-Speicher-Element (11), welches eine Flüssigkeit (M) darin speichert; und
eine Öffnereinrichtung (12), welche konfiguriert ist, um eine Öffnung (111) an dem Flüssigkeit-Speicher-Element (11) zu bilden;
einen Träger (2), welcher lösbar an dem Einweg-Flüssigkeit-Zufuhr-Modul (1) montiert ist; und
ein Zerstäubungsmodul (3), welches einen Vibrator (32), welcher an dem Träger (2) montiert ist, und eine mikroporöse Folie (31) aufweist, welche an dem Einweg-Flüssigkeit-Zufuhr-Modul (1) montiert ist und welcher lösbar an dem Vibrator (32) anliegt;
wobei das Einweg-Flüssigkeit-Zufuhr-Modul (1) eine Pufferkammer (C1) hat, welche zwischen dem Zerstäubungsmodul (3) und der Öffnung (111) des Flüssigkeit-Speicher-Elements (11) angeordnet ist, und ein Volumen der Pufferkammer (C1) geringer ist als ein Volumen der Flüssigkeit (M), welche in dem Flüssigkeit-Speicher-Element (11) gespeichert ist;
wobei das Einweg-Flüssigkeit-Zufuhr-Modul (1) konfiguriert ist, um gedrückt zu werden, sodass eine äußere Kraft auf das Flüssigkeit-Speicher-Element (11) ausgeübt wird und ein Innendruck des Flüssigkeit-Speicher-Elements (11) verändert wird, sodass ein Teil der Flüssigkeit (M) angetrieben wird, um von der Öffnung (111) in die Pufferkammer (C1) für einen Zerstäubungsprozess des Zerstäubungsmoduls (3) zu strömen;
wobei die Öffnereinrichtung (12) konfiguriert ist, um von dem Flüssigkeit-Speicher-Element (11) getrennt zu werden, indem sie relativ zu dem Flüssigkeit-Speicher-Element (11) bewegt wird, um die Öffnung (111) an dem Flüssigkeit-Speicher-Element (11) zu bilden, und wobei das Einweg-Flüssigkeit-Zufuhr-Modul (1) ferner einen Behälter (13) aufweist, welcher die Pufferkammer (C1) darin hat, und das Flüssigkeit-Speicher-Element (11) in den Behälter (13) eingesetzt ist, sodass die Öffnung (111) in räumlicher Kommunikation mit der Pufferkammer (C1) ist;
wobei der Behälter (13) einen Einlass (131) für ein Einsetzen des Flüssigkeit-Speicher-Elements (11) und einen Auslass (132) hat, welcher in Position dem Zerstäubungsmodul (3) entspricht, und wobei die Öffnereinrichtung (12) konfiguriert ist, um von dem Flüssigkeit-Speicher-Element (11) getrennt zu werden durch Zurückgehalten, indem sie in dem Einlass (131) zurückgehalten wird und relativ zu dem Flüssigkeit-Speicher-Element (11) gedreht wird, sodass die Öffnereinrichtung (12), welche von dem Flüssigkeit-Speicher-Element (11) getrennt ist, in der Pufferkammer (C1) angeordnet ist;
wobei die mikroporöse Folie (31) an dem Behälter (13) befestigt ist, und die mikroporöse Folie (31) den Auslass (132) bedeckt.

2. Zerstäubungsvorrichtung gemäß Anspruch 1, wobei der Behälter (13) eine Barriere (133) aufweist, welche in der Pufferkammer (C1) angeordnet ist und sich zwischen dem Einlass (131) und dem Auslass (132) befindet, und die Öffnereinrichtung (12), welche von dem Flüssigkeit-Speicher-Element (11) getrennt ist, an der Barriere (133) angeordnet ist durch Passieren durch den Einlass (131) hindurch.

3. Zerstäubungsvorrichtung gemäß Anspruch 1, wobei die Öffnung (111) größer als 0 mm ist und nicht größer als 8 mm ist.

## Revendications

1. Dispositif d'atomisation (100), comprenant :
un module jetable d'alimentation en liquide (1) comprenant :
un élément de stockage de liquide (11) dans lequel est stocké un liquide (M) ; et
un dispositif d'ouverture (12) configuré pour former une ouverture (111) sur l'élément de stockage de liquide (11) ;
un support (2) assemblé de manière amovible au module jetable d'alimentation en liquide (1) ; et
un module d'atomisation (3) comprenant un vibrateur (32) assemblé au support (2) et un film microporeux (31) qui est assemblé au module jetable d'alimentation en liquide (1) et qui vient en butée de manière amovible contre le vibrateur (32) ;
dans lequel le module jetable d'alimentation en liquide (1) comporte une chambre tampon (C1) disposée entre le module d'atomisation (3) et l'ouverture (111) de l'élément de stockage de liquide (11), et un volume de la chambre tampon (C1) est inférieur à un volume du liquide (M) stocké dans l'élément de stockage de liquide (11) ;
dans lequel le module jetable d'alimentation en liquide (1) est configuré pour être pressé de manière à exercer une force externe sur l'élément de stockage de liquide (11) et à modifier une pression interne de l'élément de stockage de liquide (11), de sorte qu'une partie du liquide (M) soit amenée à s'écouler depuis l'ouverture (111) jusque dans la chambre tampon (C1) pour un processus d'atomisation du module d'atomisation (3) ;
dans lequel le dispositif d'ouverture (12) est configuré pour être séparé de l'élément de stockage de liquide (11) en étant déplacé par rapport à l'élément de stockage de liquide (11) de manière à former l'ouverture (111) sur l'élément de stockage de liquide (11), et dans lequel le module jetable d'alimentation en liquide (1) comprend en outre un récipient (13) comportant la chambre tampon (C1) à son intérieur, et l'élément de stockage de liquide (11) étant inséré dans le récipient (13), de sorte que l'ouverture (111) soit en communication fluidique avec la chambre tampon (C1) ;
dans lequel le récipient (13) comporte une entrée (131) destinée à l'insertion de l'élément de stockage de liquide (11) et une sortie (132) dont la position correspond à celle du module d'atomisation (3), et dans lequel le dispositif d'ouverture (12) est configuré pour être séparé de l'élément de stockage de liquide (11) en étant retenu dans l'entrée (131) et en étant mis en rotation par rapport à l'élément de stockage de liquide (11), de sorte que le dispositif d'ouverture (12), une fois séparé de l'élément de stockage de liquide (11) soit situé dans la chambre tampon (C1) ;
dans lequel le film microporeux (31) est fixé au récipient (13), et le film microporeux (31) recouvre la sortie (132).

2. Dispositif d'atomisation selon la revendication 1, dans lequel le récipient (13) comprend une barrière (133) disposée dans la chambre tampon (C1) et située entre l'entrée (131) et la sortie (132), et dans lequel le dispositif d'ouverture (12), séparé de l'élément de stockage de liquide (11), est disposé sur la barrière (133) en passant par l'entrée (131).

3. Dispositif d'atomisation selon la revendication 1, dans lequel l'ouverture (111) est supérieure à 0 mm et n'est pas supérieure à 8 mm.
